# EUROPEAN PATENT SPECIFICATION

(11) **EP 4 471 423 B1**
(45) Date of publication and mention of the grant of the patent: **29.04.2026**
(21) Application number: 24382295.4
(22) Date of filing: 18.03.2024
(51) Int. Cl.: G01N 33/18, C12Q 1/04, F24D 17/00

(54) **METHOD FOR REAL-TIME DETERMINATION OF THE RISK INDEX FOR GROWTH OF LEGIONELLA BACTERIA IN INDOOR WATER SYSTEMS OF A BUILDING**
VERFAHREN ZUR ECHTZEITBESTIMMUNG DES RISIKO-INDEX FÜR DAS WACHSTUM VON LEGIONELLA-BAKTERIEN IN WASSERSYSTEMEN IN INNENRÄUMEN EINES GEBÄUDES
PROCÉDÉ DE DÉTERMINATION EN TEMPS RÉEL DE L'INDICE DE RISQUE POUR LA CROISSANCE DE BACTÉRIES LEGIONELLA DANS DES SYSTÈMES D'EAU INTÉRIEURE D'UN BÂTIMENT

(43) Date of publication of application: 04.12.2024
(73) Proprietor: Ulbios Techsens, S.L., 08850 Gava (Barcelona) (ES)
(72) Inventor: SANCHEZ RUIZ, LAURA, 08174 SANT CUGAT DEL VALLES (BARCELONA) (ES); GARRIDO COLOMINAS, MIGUEL ANGEL, 08022 BARCELONA (ES)
(74) Representative: Durán-Corretjer, S.L.P.

(56) References cited:
- DE-A1- 102018 122 424
- US-B1- 11 295 629
- SAETTA DANIELLA ET AL: "Data-mining methods predict chlorine residuals in premise plumbing using low-cost sensors", AWWA WATER SCIENCE, vol. 3, no. 1, 28 January 2021 (2021-01-28), XP093191778, ISSN: 2577-8161, DOI: 10.1002/aws2.1214
- FOR�T C. ET AL: "D�termination �lectrochimique de l'�paisseur des biofilms dans les circuits d'eau", MATERIAUX & TECHNIQUES, vol. 94, 2006, pages 467 - 476, XP093191513, ISSN: 0032-6895, DOI: 10.1051/mattech:2007021

## Description

The present invention belongs to the hygiene and health sector. Specifically, the present invention applies to systems that are liable to become sources of human exposure to *Legionella* bacteria in any building, with a greater potential in buildings in the hospital sector, hotel sector, geriatric residences, educational centres, sports centres and penitentiary centres.

In particular, the present invention relates to a method for determining by automatic means a risk index for the growth of *Legionella* bacteria in water networks or systems of a building. For this purpose, the method takes into account the correlation and weighting of physical, chemical and biological parameters of the water, with parameters relating to the hydraulic design of the systems and the use and structural characteristics of the building. Furthermore, the invention considers and adapts the risk assessment in real time and evaluates the corrective method to be performed if necessary.

*Legionella* is the common name for the genus *Legionella,* which is a group of Gram-negative bacteria in the form of a bacillus. These bacteria can cause an infection in humans, called legionellosis, and can present as a febrile illness, either mild or without a specific pulmonary focus (Pontiac fever), or severe as an atypical pneumonia called Legionnaires' disease. The major source of infection of these bacteria is the water system of large buildings, hotels and hospitals, humidifiers, misting machines, spas and hot springs, as well as air-conditioning systems.

The risk is such that, in 2020, Directive (EU) 2020/2184 of the European Parliament and of the Council of 16 December 2020 on the quality of water intended for human consumption was published, in which a Water Sanitation Plan (WSP) is required for existing systems in buildings in the tertiary sector, known as priority buildings. One of the most novel aspects of the regulations in this area is a new risk management methodology based on identifying and controlling the risks of this type of system. These regulations have been transposed in the 27 member states of the European Union, as in Spain, through Royal Decree RD3/2023, and in some countries this has resulted in the establishment of semi-quantitative methods for risk assessment, whereby the seriousness of the danger and the probability of the dangerous event occurring if the appropriate corrective or preventive measures are not taken must be assessed, with the risk of the existence and growth of *Legionella* being considered among the most serious. In some cases, the way in which this assessment has been performed has resulted in a risk prioritization assessment matrix. As in the following table, included in the Spanish Royal Decree RD3/2023:

**Table 1. Legionella outbreak risk matrix according to RD3/2023**

| | Insignificant | Low | Moderate | High | Very high |
|---|---|---|---|---|---|
| Very unlikely | 1 | 2 | 4 | 8 | 16 |
| Unlikely | 2 | 4 | 8 | 16 | 32 |
| Medium | 4 | 8 | 16 | 32 | 64 |
| Likely | 8 | 16 | 32 | 64 | 128 |
| Very likely | 16 | 32 | 64 | 128 | 256 |

Thus, there is a growing need to assess and obtain the risk value for the appearance or growth of *Legionella* in a building, in order to prevent an outbreak of legionellosis.

At present, the determination of the risk of *Legionella* growth in a system considered to be susceptible to the proliferation of this bacterium is based on analytical and measurement methods based on a sample of fluid from the system in question. However, numerous scientific articles and studies have shown that the risk of *Legionella* growth is associated with parameters other than water quality, such as the hydraulic design characteristics of a system, fluid temperatures, type of materials, length of the facilities, use, typology and geometry of the building, inter alia.

Examples of the influence of temperature on the presence of *Legionella* can be found in the literature in studies such as Buse et al., 2012 (Buse, H. Y., Schoen, M. E., and Ashbolt, N. J. (2012). Legionellae in engineered systems and use of quantitative microbial risk assessment to predict exposure. Water Research, 46, 921-933*),* Marchesi et al., 2011 (Marchesi, I., Marchegiano, P., Bargellini, A., Cencetti, S., Frezza, G., Miselli, M., & Borella, P. (2011). Effectiveness of different methods to control Legionella in the water supply: ten-year experience in an Italian university hospital. Journal of Hospital Infection, 77, 47-51*),* Mouchtouri et al., 2007 *(*Mouchtouri, V., Velonakis, E., & Hadjichristodoulou, C. (2007). Thermal disinfection of hotels, hospitals, and athletic venues hot water distribution systems contaminated by Legionella species. American Journal of Infection Control, 35, 623-627*)* o Ndiongue et al., 2005 *(*Ndiongue, S., Huck, P. M., & Slawson, R. M. (2005). Effects of temperature and biodegradable organic matter on control of biofilms by free chlorine in a model drinking water distribution system. Water Research, 39, 953-964*).*

As regards the influence of other parameters such as pipe length and stagnation, there is a broad consensus on the impact these have on the precision, accuracy and efficiency of the ways of estimating the risk of *Legionella* growth. However, few studies have empirically evaluated these factors in practice. Some studies have provided information on the parameters considered to be the best predictors of *Legionella* contamination risk, such as Eboigbodin et al., 2008 *(*Eboigbodin, K. E., Seth, A., and Biggs, C. A. (2008). A review of biofilms in domestic plumbing. Journal of the American Water Works Association, 100,131-138*)* o Volker et al., 20016 (Volker, S., Schreiber, C. Kistemann, T. (2016) Modelling characteristics to predict Legionella contamination risk - Surveillance of drinking water plumbing systems and identification of risk areas. International Journal of Hygiene and Environmental Health, 219, 101-109*).* Estudios como el de De Giglio et al., 2019 *(*De Giglio, O. (2019) Legionella and legionellosis in touristic-recreational facilities: Influence of climate factors and geostatistical analysis in Southern Italy (2001-2017) Environmental Research, 178, 108721)*,* have even taken into account parameters such as climatic influence.

US11295629 discloses determining the risk for growth of Legionella in a water system, by measuring two values, one being a temperature and the other being a bioactivity measured by a protein sensor. The bioactivity value is compared to a threshold limit.

In other words, the prior art clearly identifies which are the different parameters that impact on *Legionella* development. However, despite having correctly identified the main parameters, the risk indices calculated therefrom do not predict the existence and growth of *Legionella* with the desirable accuracy as they do not take into account the changes that occur in the systems over time, or base their predictions on microbiological controls that do not give a real risk value (*De Giglio, O. (2019)).* To date, there is no standardized system or method that simultaneously and periodically measures and evaluates all the determining factors for knowing the risk of *Legionella* growth in the systems of a building that use or may use water as a transmission vector, enabling the prediction, control and early prevention of nosocomial and/or environmental infections along with the corrective measures to be employed. There is thus a need in the sector to improve the determination of predictive indices of risk of *Legionella* growth, on the basis of which to take the necessary preventive measures. The present invention meets this need.

The present invention provides a method for the determination via automated means of a *Legionella* growth risk index in a water system of a building, said risk index being expressed as a numerical value, comprising the following steps:
I) measurement and storage in an electronic memory of at least three values corresponding to variables selected from the group comprising physical, physicochemical and biological features of the water system; each of these values corresponding to a risk value; and
II) calculating the overall risk index via automated means from the values by weighted composition; wherein
   the method comprises measuring bioactivity in the water system by means of at least one biosensor placed in the water system,
   determining via automated means the difference between an expected and an actually measured bioactivity value
   and in that, in the calculation of the risk index in step II, the abovementioned difference is used as one of the abovementioned measured and stored values from step I to determine the risk index.

Preferably, the method comprises measuring and storing at least 4, at least 5, at least 6, at least 7, at least 8, at least 9, or at least 10 different values in step l. Preferably, the number of variables measured and stored in step I is as large as possible.

In a preferred embodiment, said automated means are processors programmed to perform different operations such as those selected from the group comprising accessing the values recorded in the memory, calculating the difference between expected and measured bioactivity values and calculating the overall risk index by weighted composition.

The values corresponding to physical parameters refer to features and hydraulic design of a facility and geometrical parameters of the building. Thus, in accordance with the invention, the method comprises among the measured values of step I, characteristic values of the system selected from a group comprising: the age of the system, the material of the system, the existence and typology of recirculation of the system, the distribution network and the use of water storage tanks.

After studying the data obtained in more than 900 buildings in the tertiary sector of various typologies, over a period of 8 years, technical publications and scientific papers, in which the characteristics and hydraulic design of a system and geometric parameters of the building have been compiled, the inventors have come to the conclusion that there is a need for a method for the determination of a *Legionella* growth risk index as defined by the present invention, which is capable of taking into account physical, physicochemical and/or biological features of the system and which includes the prediction of the growth of the system's own biofilm in the calculation of the risk index. Knowing the bioactivity data in the water system, the biofilm growth cycle is understood, the method allows morphological filters to be applied to assess the growth risk, for example by means of a baseline biofilm growth curve, to identify growth trends over a duration attributable to a *Legionella* growth risk.

In a preferred embodiment, the method comprises automated means for analysing and defining bioactivity within the water system based on measurements of the at least one biosensor performed over at least a period of one month. This would allow the method to take a sufficient number of measurements to perform a correct analysis on the bioactivity and not to lose information. More preferably, the number of measurements analysed during the one-month period is at least 2800 measurements. Even more preferably, the number of measurements analysed during the period of one month is between 2800 and 3000 measurements.

Preferably, the present invention comprises automated means for assessing the overall biofilm growth trend and/or the medium-term growth trend and/or the short-term growth trend, by analysing successive bioactivity measurements by means of the biosensors. Preferably, said automated means are programming environments and specialized software for the creation, implementation and analysis of mathematical models. More preferably, such automated means make use of a programming and numerical computing platform for data analysis, algorithm development, mathematical modelling, simulation and visualization of the above. Preferably, the overall growth trend is measured from the point of origin. More preferably, the origin point is the first day of monitoring or when any event considered as a new origin point occurs. Preferably, a point of origin can be after performing a corrective shock treatment on the system. Preferably, the medium-term growth trend is that obtained from measurements taken over 60 days. Also preferably, the short-term growth trend is that obtained from measurements taken over 30 days.

In a preferred embodiment, the method comprises measuring the temperature by means of at least one temperature sensor placed in the system and using it to determine the bioactivity value via automated means. Preferably, the at least one temperature sensor is installed in the same location as the at least one biosensor.

More preferably, the method comprises measuring the redox potential (ORP) by means of at least one ORP sensor placed in the system and using it in the determination of the bioactivity value by said automated means. Preferably, said sensor is installed at the point in which the last disinfection treatment of the system is performed.

In another preferred embodiment, the method comprises both measurements not only of the redox potential (ORP) but also of the temperature by means of at least one sensor for each installed in the water system and their use in the determination of the bioactivity value via automated means. Preferably, the at least one ORP sensor is installed at the point where the last disinfection treatment of the system takes place. Preferably, the at least one temperature sensor is installed at the same location as the at least one biosensor.

There are physicochemical factors corresponding to the water quality that can affect the determination of the *Legionella* growth risk index. Thus, in a preferred embodiment, the method makes use of measurements in step I, which comprise measuring at least two risk values by means of at least one sensor installed in the water system, from the values selected from the group comprising: temperature, amount of total chlorine, amount of free chlorine, pH and the redox potential (ORP).

Each measured value is a value that is weighted by automated means on the basis of an intrinsic database. Preferably, said database comprises data obtained from studies of water systems and knowledge of the prior art. In a preferred embodiment, said database has data specific to the system in which the risk is being assessed. More preferably, said database has a history of data from the system, taking into account the historical behaviour, in terms of *Legionella,* of *Legionella* positives or outbreaks in the system, their repetition over time and their improvement or otherwise depending on the corrective treatment performed.

Preferably, the method comprises automated means for updating its database with the values resulting from measurements of the water system for which the risk index for growth of *Legionella* is being determined. Even more preferably, said database is automatically updated with each measurement performed, creating a history.

In a preferred embodiment, the method comprises automated means for adjusting the weighting of the given risk value to a value measured in step I according to its lower and/or upper threshold value. At the present time, there are defined threshold values for some of the abovementioned risk values, but each system has its own threshold values. Preferably, the method comprises automated means for determining the threshold values of the measured risk values. Thus, in those embodiments in which the method adjusts the threshold of each value, it is better adapted to the system. Preferably, at least to a first approximation, the lower threshold value of each measured risk value is the mean value thereof minus three times the standard deviation and/or the upper threshold value of each measured risk value is the mean value thereof plus three times the standard deviation.

More preferably, the method takes into account the existing correlation of considered risk values or factors and, as such, a preferred embodiment of the method comprises automated means for adjusting the dependence between different measured risk values. Preferably, the automated means model the incidence between the measured risk values and disregard the dependent values based on the dependence percentages obtained.

In a preferred embodiment, the risk index calculation is performed by automated means applying generalized linear mathematical models (GLM) based on multivariate strategies and the risk index entailed by the growth and exposure of bacteria in human water systems using the Quantitative Microbial Risk Assessment (QMRA) methodology.

This QMRA methodology is included in the World Health Organization (WHO) guidelines since 2016 as Quantitative Microbial Risk Assessment: application to water safety management. It is based on a four-step risk assessment process:
(1) Identification of the potential hazard to humans
(2) Exposure assessment
(3) Dose-response analysis: Determination of the probability of infections via mathematical modelling
(4) Risk characterization.

In a preferred embodiment, the present invention comprises automated means, such as software that allows a user to view, manipulate and enter values. In another preferred embodiment, the method comprises automated means that inform the user of the risk index for growth of *Legionella.* In a preferred embodiment, the automated means informs the user whether the value of the risk index for growth is so high as to require corrective action to be taken, after comparing that value with the risk values assigned according to the reference risk matrix. Preferably, such corrective measures comprise cleaning of the water system. Preferably, the method comprises automated means for indicating the effectiveness of a corrective measure performed. In a preferred embodiment, the method comprises the use of automated means allowing a user to access such information. More preferably, such automated means are the set of programs included in a computer. Even more preferably, the present invention comprises automated means for adjusting the risk values that are assigned to each measured value according to a considered risk matrix. Preferably, said risk matrix adjusts the weightings of the risk values on the basis of a specific standard.

Preferably, the method according to any of the abovementioned embodiments refers to a method for determining the risk index for growth of *Legionella* in a cold water system or network of a building. In another preferred embodiment, the method refers to a method for determining the risk index for growth of *Legionella* in a domestic hot water (DHW) system or network of a building. In another preferred embodiment, the method refers to a method for determining the risk index for growth of *Legionella* in both water systems or networks.

The present invention is novel and advantageous in that it counts the difference between the expected bioactivity value in the water system with respect to the measured value as a further risk value to be considered together with other measurements in order to determine a risk index for growth of *Legionella* more accurately than the methods included in the prior art. Furthermore, the method allows the expected value to be adjusted based on the measurements made and also to weight each variable based on the knowledge of the prior art in contrast with a history of its own data, taking into account the parameters recognized as fundamental and the behaviour and history of the systems in which it is going to be applied, its repetition over time and its improvement or otherwise depending on the corrective treatment performed. In addition, preferred embodiments of this method allow the bioactivity risk values to be adjusted on the basis of the measurement of other variables that may influence bioactivity, such as temperature and/or redox potential (ORP).

The method described takes into account the effect of the risk values and how their weight has a direct impact on the existing risk index, and also their evolution over time, having been underestimated hitherto in the risk assessment methodology. As such, this is a unique invention that innovates in the estimation of the risk of outbreaks of nosocomial and environmental legionellosis, which encompasses the weighting of factors and their evolution over time.

As a fundamental point, the method described determines the risk index for growth of *Legionella* in the system considered, beyond the information provided by means of prediction techniques and physical analysis techniques, with the aim of being able to act prior to a possible health alarm.

It is understood that, for a person skilled in the art, any of the various embodiments described above that make up the present invention can be combined with each other.

In the context of the present invention, the concept "variable", "parameter" or "factor" refers to a magnitude that can be measured or quantified in the context of a system, process or installation, being essential for the analysis and control of such systems. The term 'risk value' refers to the value assigned to a 'variable', 'parameter' or 'factor' considered as a risk variable for *Legionella* growth, to be used *a posteriori* in the calculation of the risk index by weighted composition.

In the field of physicochemical risk values, the term "physicochemical variable" is understood in the present invention to mean those magnitudes involving physical or chemical properties, such as temperature, concentration of chemical substances, conductivity, inter alia, which are relevant to the functioning of the water system.

In the field of physicochemical or biological growth measurements, it is understood that methods for determining measurement variables in water refer to specific processes for quantifying parameters in water. Such methods may refer to the use of "physicochemical sensors" and "biosensors", which entail devices that are capable of measuring and detecting physical, chemical or biological quantities in the system, respectively. Such methods may also refer to measurements derived from sampling and analysis or culturing of samples in laboratories. In the context of the present invention, the term "sensors" refers to devices that are capable of measuring physical or chemical quantities in the system, providing quantitative information about relevant variables. These sensors are essential for system monitoring and control, enabling real-time data collection for informed decision making. It is to be understood that the sensors referred to in the present invention have automated means for telematically sending the data of the measurements they perform, which are recorded in an electronic memory and processed by other automated means, capable of using each measured value and calculating the risk index as described in the present invention.

In addition, "bioactivity sensors" or "biosensors" are a specific category of sensors that are capable of measuring and detecting quantities associated with biological processes. These devices are designed to detect and measure biological quantities, for instance the presence of microorganisms or specific biomolecules such as ATP or polysaccharide and protein content, bioactivity, for example through the electrochemical biofilm signal, or the amount of biomass. In the context of the present invention, biosensors are crucial tools for monitoring relevant biological activity or bioactivity in water systems. A biofilm sensor or sensor for monitoring biofilm growth is considered a type of biosensor or bioactivity sensor. An example of a biofilm monitoring sensor that measures bioactivity (i.e. a biosensor) is described by Pavanello, G., Faimali, M., Pittore, M., Mollica, A., Mollica, A., and Mollica, A. (2011). Exploiting a new electrochemical sensor for biofilm monitoring and water treatment optimization. Water Research, 45(4). Specifically, this biosensor example performs bioactivity measurements indirectly by means of potentiostatic and intensiostatic measurements and records the bioactivity as BES (biofilm electrochemical signal), expressed as a current density or potential (mV vs Zn). In particular, in the present invention, this type of biosensor is used to perform bioactivity measurement.

The term "biofilm" in this document refers to a layer formed by microorganisms attached to the surfaces of the internal water systems of a building, which is relevant in the context of the present invention, which seeks to understand and monitor their growth in order to calculate the risk value associated with such growth.

In the present invention, the term "morphological filters" refer to methods of processing data or images that alter their shape or structure, being used for specific analysis of biofilm growth.

In the context of the present invention, the term "automated means" refers to devices, systems or processes that operate in an automated manner, i.e. without direct human intervention. These means may include electronic components, software, sensors, actuators or other technologies that enable the automatic execution of specific functions within the patented system.

In situations related to cases of *Legionella* in water systems of buildings, the "corrective treatments" mentioned in the present invention are defined as actions or processes applied to address and correct situations considered to be risks, specifically related to the degree of presence or possibility of presence of *Legionella.* An example of corrective action would comprise cleaning and/or disinfection of the water system.

In the present invention, the term "weighting" refers to the process of assigning relative values to different variables or factors according to their importance in the system, which is essential for informed decision making.

Weighting has a specially designed network of correlations to match the value to a large number of variables that will increase the accuracy and precision of the results as it is used, ranging from weighted compositions to complex equations that are conditioned by the input values.

Weighted compositions are mathematical functions such as a weighted average, in which a value is obtained, in this case the risk index, by multiplying each of the data (risk value assigned to the measured values) by its weight and then adding them together, thus obtaining a weighted sum; it is then divided by the sum of the weights, resulting in the weighted average, or risk index.

Complex equations are understood as the relationships established between different parameters that, directly or indirectly, make it possible to obtain, as if they were digital twins, in some cases, quantitative indicators in order to obtain numerical evaluations from different dimensions of the system. The parameterization functions of the different variables depend on the dimension that different subsections - or calculation levels - deal with, and it is possible to find the same variable in different calculation levels, both for the same equation and for different correlations. Among the different operators, there are multiplications, conditionals and qualitative to quantitative signal converters.

In the context of the present invention, the term "threshold value" refers to a minimum or maximum value of a magnitude beyond which a given effect occurs, namely the minimum or maximum value of a variable beyond which its value changes in the weighting to determine the risk index of the system.

In the context of the present invention, the term "mathematical model" refers to an abstract and structured representation of a system or process by means of mathematical equations and relationships. These models seek to describe and predict the behaviour of the system, facilitating analysis and decision making. In the context of the present invention, mathematical models are essential tools for understanding and optimizing the performance of the systems and processes involved.

The acronym "QMRA" stands for "Quantitative Microbial Risk Assessment". In the context of the present invention, QMRA is a systematic approach that uses mathematical modelling to quantify the risks associated with the presence of microorganisms, such as *Legionella,* in water systems. It enables accurate assessment and management of microbiological risks to humans in the context of the patent.

The acronym "GLM" stands for "Generalized Linear Model". In the patent, GLM is a type of mathematical model that is used to analyse relationships between variables, even when the distribution of data does not follow a normal distribution. This approach is valuable for understanding and modelling complex phenomena present in the present invention, considering different types of parameters and non-linear relationships.

The "geometrical variables" in the present invention are quantities related to the dimensions and shapes of the pipes and of the water system.

When the "design of a system" is mentioned in the present invention, it refers to the process of planning and configuring a system or installation, considering various variables for its optimal functioning.

In the present invention, the "hydraulic design of a system" refers to the specific planning of hydraulic aspects within the system, such as those aspects that allow for the flow or stagnation of water and control thereof.

For the purpose of better understanding, a number of figures related to a possible embodiment of the method performed in accordance with the present invention are attached hereto, by way of non-limiting explanation.
Figure 1 shows the analysis over time of the bioactivity, the measurement of temperature and redox potential (ORP) values and the determination of thresholds for the latter two during the period of 21 000 (2.1 x 10⁴) measurements of an embodiment of the present invention in a specific building and system.
Figure 2 shows an example of the same embodiment of figure 2 during the period of 23 000 (2.3 x 10⁴) measurements.
Figure 3 shows an example of the same embodiment of figures 2 and 3 during the period of 25 000 (2.5 x 10⁴) measurements.
Figure 4 shows the study of the dependence between detailed variables and statistical results for consideration or discarding.

A non-limiting example of the method of the present invention comprises automated means such as a computer in which a user can select and/or adjust the ranges of the measured values so that the risk index for growth of *Legionella* is obtained within the framework of said regulation.

Thus, in said non-limiting example, a user can enter the values due to intrinsic characteristics of the water system of the building where the method is being applied, such as physical parameters related to the characteristics and hydraulic design of the system and geometrical parameters of the building. Furthermore, in this embodiment, the method comprises the measurement of other bioactivity and physicochemical variables via the use of sensors placed in the system or water network of the building, which collect the measurement of values that are sent telematically to the same computer. This computer has automated means for recording the measured values, and automated means, such as a programming and numerical computing platform for data analysis, algorithm development, creation of mathematical models, simulation and visualization of the above, to analyse the growth of the biofilm in the system by virtue of the bioactivity measurements, calculate the difference between the expected growth value and the measured value and use it as a risk value, which will be used to determine the *Legionella* risk index together with the rest of the measured values. In this way, the risk index is a value calculated and adjusted for each measurement, which will warn the user of the need to take corrective measures if it approaches high risk values, according to the risk matrix entered. In the event of exceeding a risk value considered as high, the system warns the user to take corrective preventive measures, for instance cleaning and/or disinfection of the water system.

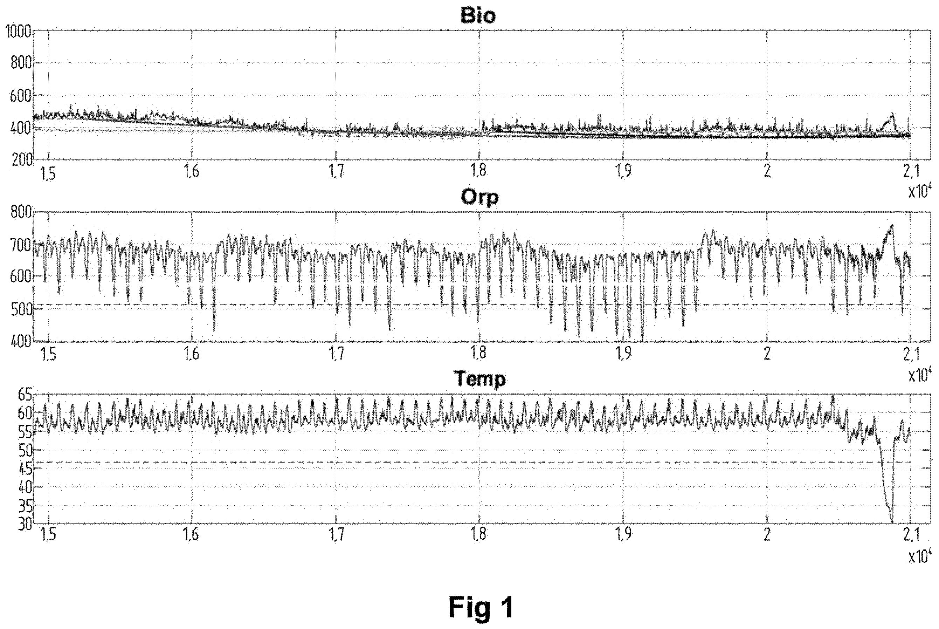

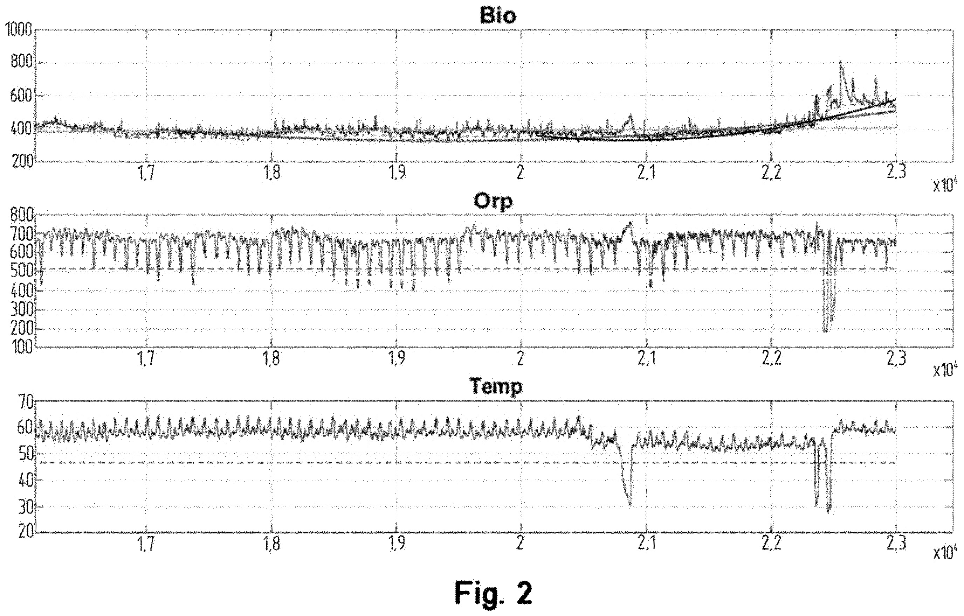

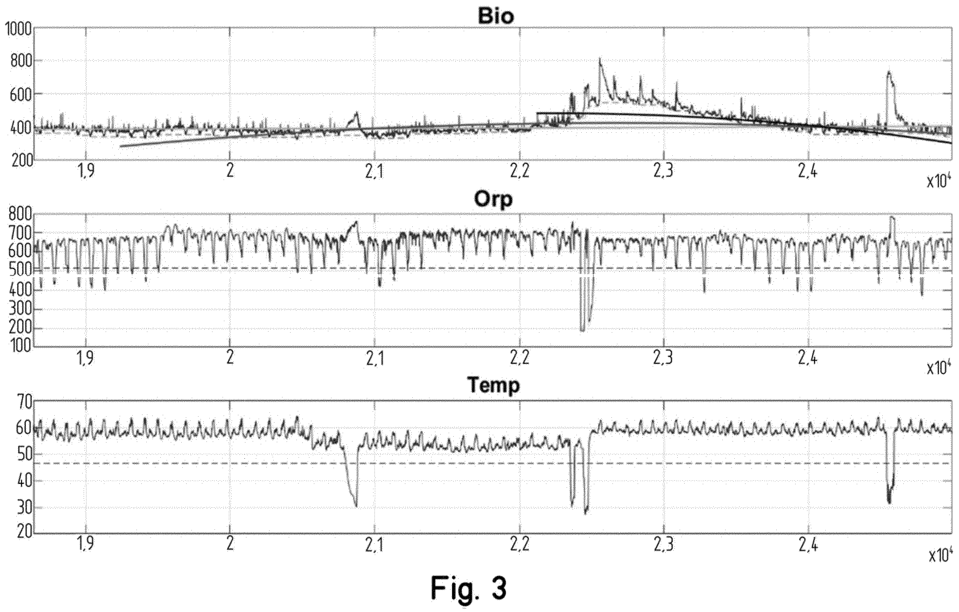

Figures 1, 2 and 3 are examples showing the periodic measurement of bioactivity, temperature and redox potential (ORP) risk values for this same embodiment of the method. Specifically, the three figures show the evolution over time of the risk values taken from 21 000 (2.1 x 10⁴) data in figure 1, from 23 000 (2.3 x 10⁴) data in figure 2, and from 25 000 (2.5 x 10⁴) data in figure 3. In these figures, it is possible to observe how the threshold values (dashed lines) of the temperature and redox potential values vary as the amount of data increases. Furthermore, the three figures show an embodiment of the method comprising automated means for displaying a first graph (Bio) corresponding to the bioactivity sensor data for monitoring biofilm growth in the system, and in which three different curves are observed, resulting from the application of morphological filters by the automated means to identify growth trends according to the bioactivity data measured at overall, medium-term and short-term levels.

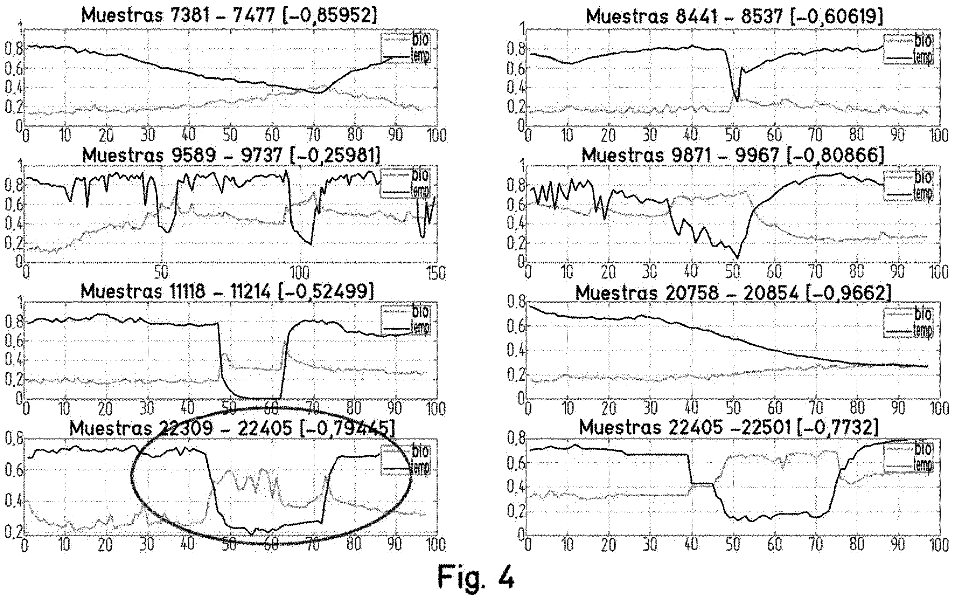

Figure 4 shows how the same example of the invention comprises automated means to represent an analysis on the dependence between measured values. This embodiment of the method comprises automated means for modelling the incidence between values and disregarding the dependent parameters on the basis of the percentage of dependence in order to detail the degree of incidence between risk values and obtain a more accurate risk index. In a first approximation, when the percentage of dependency is greater than 75%, the resulting data are disregarded as valid data for the calculation of the risk index and are not considered. Specifically, the figure shows that 79.4% (result -0.79445) of the bioactivity measurement is given by the temperature variable in this period, which means that these bioactivity data should not be considered as risk as they are the result of the dependence of another variable.

The inventors have tested the method described by the present invention in several buildings and demonstrated that it works. When the bioactivity value determined by the bioactivity sensor was higher or lower than expected, the index value was adjusted, and when the index value exceeded an acceptable index value according to the risk framework (e.g. a state regulation) to which it was adjusted, appropriate preventive and/or corrective measures were taken, for instance cleaning and/or disinfection of the system, allowing biofilm growth to be controlled and preventing outbreaks of legionellosis.

With all these data, the example method obtains a series of risk values with which to make a weighted composition to calculate the growth index for *Legionella* in the water system. The method thus comprises the measurement of values, the adjustment of these risk values based on the threshold value of each variable and the compensation of these values when they are dependent. Furthermore, in order to be able to calculate the risk value due to bioactivity in the water system, the method comprises analysing the bioactivity measurements obtained by a biosensor via automated means to determine the expected biofilm growth and comparing it with the measured data.

Although the invention has been described and illustrated with reference to a number of representative examples, it will be appreciated that this exemplary embodiment is in no way limiting on the present invention and therefore any of the variants included within the content of the appended claims are to be considered as included within the scope of the present invention.

## Claims

1. Method for determining the risk index for growth of *Legionella* in the water systems of a building, said risk index being expressed as a numerical value, comprising the following steps:
I) measurement and storage in an electronic memory of values corresponding to variables selected from the group comprising physical, physicochemical and biological features of said water system, each of said values corresponding to a risk value; and
II) calculation of the overall risk index via automated means from the values by weighted composition;
the method comprises the measurement of bioactivity in the water system by means of at least one biosensor installed in said water system,
**characterized in that**
the determination via automated means of the difference between an expected bioactivity value and the value actually measured by the biosensor is used as one of the abovementioned measured and stored values from step I to determine the risk index; and **in that**
at least three values are measured in step I, wherein the method comprises among the measured values of step I, characteristic values of the system selected from a group comprising: the age of the system, the material of the system, the existence and typology of recirculation of the system, the distribution network and the use of water storage tanks.

2. Method according to Claim 1, wherein the method comprises measuring the temperature by means of at least one temperature sensor installed in the system and using it to determine the bioactivity value via automated means.

3. Method according to Claim 2, wherein said sensor is in the same location as the at least one biosensor.

4. Method according to any one of Claims 1 to 3, wherein the method comprises measuring the redox potential (ORP) by means of at least one ORP sensor installed in the water system and using it in the determination of the bioactivity value via automated means.

5. Method according to Claim 4, wherein said sensor is located at the point where the last disinfection treatment of the system is performed.

6. Method according to any one of the preceding claims, wherein the measurement of step I comprises measuring at least two variables by means of at least one sensor installed in the water system, of the variables selected from the group comprising: temperature, amount of total chlorine, amount of free chlorine, pH, and redox potential (ORP).

7. Method according to any one of the preceding claims, wherein the method comprises automated means for determining the threshold values of the values measured in step I.

8. Method according to Claim 7, wherein the lower threshold value of each value is the mean value thereof minus three times its standard deviation and the upper threshold value of each value is the mean value thereof plus three times its standard deviation.

9. Method according to Claim 7 or 8, wherein the method comprises automated means for adjusting the weighting of the measured values according to their threshold values.

10. Method according to any one of the preceding claims, wherein the method comprises automated means for adjusting the dependence between the different measured values.

11. Method according to any one of the preceding claims, wherein the measurement of step I comprises measuring at least two characteristic risk values of the installation selected from a group comprising: the age of the installation, the material of the installation, the existence and typology of recirculation of the system, the distribution network and the use of water storage tanks.

12. Method according to any one of the preceding claims, wherein the method comprises automated means for assigning base weights to each measured value in step I on the basis of intrinsic data obtained from studies of water systems and knowledge of the prior art.

13. Method according to Claim 12, wherein the method comprises automated means for updating its database with the values resulting from measurements of the water system for which the risk index for growth of *Legionella* is being determined.

14. Method according to any one of the preceding claims, wherein the method relates to a method for determining the risk index for growth of *Legionella* in a domestic hot water (DHW) system of a building.

## Patentansprüche

1. Verfahren zum Bestimmen des Risikoindexes für das Wachstum von Legionellen in den Wassersystemen eines Gebäudes, wobei der Risikoindex als numerischer Wert ausgedrückt wird, umfassend die folgenden Schritte:
I) Messen und Speichern von Werten, die Variablen entsprechen, die aus der Gruppe ausgewählt sind, die physikalische, physikalisch-chemische und biologische Merkmale des Wassersystems umfasst, wobei jeder dieser Werte einem Risikowert entspricht; und
II) Berechnung des Gesamtrisikoindexes durch automatisierte Mittel aus den Werten durch gewichtete Zusammensetzung;
wobei das Verfahren die Messung der Bioaktivität im Wassersystem mittels mindestens eines in dem Wassersystem installierten Biosensors umfasst,
**dadurch gekennzeichnet, dass**
die Bestimmung der Differenz zwischen einem erwarteten Bioaktivitätswert und dem tatsächlich vom Biosensor gemessenen Wert durch automatische Mittel als einer der oben genannten gemessenen und gespeicherten Werte aus Schritt I zur Bestimmung des Risikoindex verwendet wird; und dadurch, dass
in Schritt I mindestens drei Werte gemessen werden, wobei
das Verfahren unter den gemessenen Werten von Schritt I charakteristische Werte des Systems umfasst, die aus einer Gruppe ausgewählt sind, die Folgendes umfasst: das Alter des Systems, das Material des Systems, das Vorhandensein und die Typologie der Rezirkulation des Systems, das Verteilungsnetz und die Verwendung von Wasserspeichertanks.

2. Verfahren nach Anspruch 1, wobei das Verfahren das Messen der Temperatur mittels mindestens eines im System installierten Temperatursensors und die Verwendung dieser Temperatur zur Bestimmung des Bioaktivitätswerts durch automatisierte Mittel umfasst.

3. Verfahren nach Anspruch 2, wobei sich der Sensor an derselben Stelle wie der mindestens eine Biosensor befindet.

4. Verfahren nach einem der Ansprüche 1 bis 3, wobei das Verfahren das Messen des Redoxpotentials (ORP) mittels mindestens eines im Wassersystem installierten ORP-Sensors und dessen Verwendung zur Bestimmung des Bioaktivitätswerts durch automatisierte Mittel umfasst.

5. Verfahren nach Anspruch 4, wobei sich der Sensor an der Stelle befindet, an der die letzte Desinfektionsbehandlung des Systems durchgeführt wird.

6. Verfahren nach einem der vorstehenden Ansprüche, wobei die Messung in Schritt I das Messen von mindestens zwei Variablen mittels mindestens eines im Wassersystem installierten Sensors umfasst, wobei die Variablen aus der Gruppe ausgewählt sind, die Folgendes umfasst: Temperatur, Gesamtchlorgehalt, Gehalt an freiem Chlor, pH-Wert und Redoxpotenzial (ORP).

7. Verfahren nach einem der vorstehenden Ansprüche, wobei das Verfahren automatisierte Mittel zum Bestimmen der Schwellenwerte der in Schritt I gemessenen Werte umfasst.

8. Verfahren nach Anspruch 7, wobei der untere Schwellenwert jedes Wertes dessen Mittelwert minus dem Dreifachen seiner Standardabweichung ist und der obere Schwellenwert jedes Wertes dessen Mittelwert plus dem Dreifachen seiner Standardabweichung ist.

9. Verfahren nach Anspruch 7 oder 8, wobei das Verfahren automatisierte Mittel zum Anpassen der Gewichtung der gemessenen Werte entsprechend ihren Schwellenwerten umfasst.

10. Verfahren nach einem der vorstehenden Ansprüche, wobei das Verfahren automatisierte Mittel zum Anpassen der Abhängigkeit zwischen den verschiedenen Messwerten umfasst.

11. Verfahren nach einem der vorstehenden Ansprüche, wobei die Messung in Schritt I das Messen von mindestens zwei charakteristischen Risikowerten der Anlage umfasst, die aus einer Gruppe ausgewählt sind, die Folgendes umfasst: das Alter der Anlage, das Material der Anlage, das Vorhandensein und die Typologie der Rückführung des Systems, das Verteilungsnetz und die Verwendung von Wasserspeichertanks.

12. Verfahren nach einem der vorstehenden Ansprüche, wobei das Verfahren automatisierte Mittel zum Zuweisen von Basisgewichten zu jedem in Schritt I gemessenen Wert auf der Grundlage von intrinsischen Daten umfasst, die aus Studien zu Wassersystemen und dem Stand der Technik gewonnen wurden.

13. Verfahren nach Anspruch 12, wobei das Verfahren automatisierte Mittel zum Aktualisieren seiner Datenbank mit den Werten umfasst, die sich aus Messungen des Wassersystems ergeben, für das der Risikoindex für das Wachstum von Legionellen bestimmt wird.

14. Verfahren nach einem der vorstehenden Ansprüche, wobei sich das Verfahren auf ein Verfahren zur Bestimmung des Risikoindex für das Wachstum von Legionellen in einem Warmwassersystem (DHW) eines Gebäudes bezieht.

## Revendications

1. Procédé pour déterminer l'indice de risque de croissance de *Legionella* dans les systèmes d'eau d'un bâtiment, ledit indice de risque étant exprimé sous forme de valeur numérique, comprenant les étapes suivantes consistant à :
i) mesurer et stocker dans une mémoire électronique de valeurs correspondant à des variables sélectionnées parmi le groupe comprenant les caractéristiques physiques, physicochimiques et biologiques dudit système d'eau, chacune desdites valeurs correspondant à une valeur de risque ; et
ii) calculer l'indice de risque global par des moyens automatisés à partir des valeurs par composition pondérée ; le procédé comprend la mesure de la bioactivité dans le système d'eau au moyen d'au moins un biocapteur installé dans ledit système d'eau,
**caractérisé en ce que**
la détermination par des moyens automatisés de la différence entre une valeur de bioactivité attendue et la valeur réellement mesurée par le biocapteur est utilisée comme l'une des valeurs mesurées et stockées susmentionnées de l'étape l pour déterminer l'indice de risque ; et **en ce que**
au moins trois valeurs sont mesurées à l'étape l, dans lequel
le procédé comprend, parmi les valeurs mesurées à l'étape l, des valeurs caractéristiques du système sélectionnées parmi un groupe comprenant : l'âge du système, le matériau du système, l'existence et la typologie de la recirculation du système, le réseau de distribution et l'utilisation de réservoirs de stockage d'eau.

2. Procédé selon la revendication 1, dans lequel le procédé comprend la mesure de la température au moyen d'au moins un capteur de température installé dans le système et son utilisation pour déterminer la valeur de bioactivité par des moyens automatisés.

3. Procédé selon la revendication 2, dans lequel ledit capteur se trouve au même endroit que ledit au moins un biocapteur.

4. Procédé selon l'une quelconque des revendications 1 à 3, dans lequel le procédé comprend la mesure du potentiel redox (ORP) au moyen d'au moins un capteur ORP installé dans le système d'eau et son utilisation dans la détermination de la valeur de bioactivité par des moyens automatisés.

5. Procédé selon la revendication 4, dans lequel ledit capteur est situé au point où le dernier traitement de désinfection du système est effectué.

6. Procédé selon l'une quelconque des revendications précédentes, dans lequel la mesure de l'étape l comprend la mesure d'au moins deux variables au moyen d'au moins un capteur installé dans le système d'eau, parmi les variables choisies dans le groupe comprenant : la température, la quantité de chlore total, la quantité de chlore libre, le pH et le potentiel redox (ORP).

7. Procédé selon l'une quelconque des revendications précédentes, dans lequel le procédé comprend des moyens automatisés pour déterminer les valeurs seuils des valeurs mesurées à l'étape l.

8. Procédé selon la revendication 7, dans lequel la valeur seuil inférieure de chaque valeur est la valeur moyenne de celle-ci moins trois fois son écart type et la valeur seuil supérieure de chaque valeur est la valeur moyenne de celle-ci plus trois fois son écart type.

9. Procédé selon la revendication 7 ou 8, dans lequel le procédé comprend des moyens automatisés pour ajuster la pondération des valeurs mesurées en fonction de leurs valeurs seuils.

10. Procédé selon l'une quelconque des revendications précédentes, dans lequel le procédé comprend des moyens automatisés pour ajuster la dépendance entre les différentes valeurs mesurées.

11. Procédé selon l'une quelconque des revendications précédentes, dans lequel la mesure de l'étape l comprend la mesure d'au moins deux valeurs de risque caractéristiques de l'installation choisies parmi un groupe comprenant : l'âge de l'installation, le matériau de l'installation, l'existence et la typologie de la recirculation du système, le réseau de distribution et l'utilisation de réservoirs de stockage d'eau.

12. Procédé selon l'une quelconque des revendications précédentes, dans lequel le procédé comprend des moyens automatisés pour attribuer des pondérations de base à chaque valeur mesurée à l'étape l sur la base de données intrinsèques obtenues à partir d'études sur les systèmes d'eau et de connaissances de l'état de la technique.

13. Procédé selon la revendication 12, dans lequel le procédé comprend des moyens automatisés pour mettre à jour sa base de données avec les valeurs résultant des mesures du système d'eau pour lequel l'indice de risque de croissance de Legionella est déterminé.

14. Procédé selon l'une quelconque des revendications précédentes, dans lequel le procédé concerne un procédé pour déterminer l'indice de risque de croissance de *Legionella* dans un système d'eau chaude sanitaire (ECS) d'un bâtiment.
